# EUROPEAN PATENT APPLICATION

(11) **EP 2 135 601 A1**
(43) Date of publication of application: **23.12.2009**
(21) Application number: 08158678.6
(22) Date of filing: 20.06.2008
(51) Int. Cl.: A61K 9/16, A61K 31/496, A61K 31/41

(54) **Stabilization of amorphous drugs using sponge-like carrier matrices**

(71) Applicant: Capsulution Nanoscience AG, 12489 Berlin (DE)
(72) Inventor: González Ferreiro, María, 10119 Berlin (DE); Dunmann, Christoph, 12435 Berlin (DE); Kröhne, Lutz, 10439 Berlin (DE); Voigt, Andreas, Dr., 12621 Berlin (DE)
(74) Representative: Kilger, Ute

(57) **Abstract**

The present invention relates to drug formulations for the stabilization of amorphous forms of drugs. In particular the present invention relates to pharmaceutical compositions comprising sponge-like carrier matrices, particularly polyelectrolyte complexes or porous particles. The invention also relates to methods for the production of such pharmaceutical compositions.

## Description

### Technical field of the invention

The present invention is in the field of drug formulation. In particular the present invention relates to the stabilization of the amorphous state of drugs using sponge-like carrier matrices.

### Background of the invention

The enhancement of dissolution and bioavailability is desired for drugs with low solubility. Amorphous forms of drugs often have a higher bioavailability than crystalline forms of the same drug. The crystalline form of the solid state of poorly water-soluble components influences both, the dissolution kinetics and the limiting drug concentration. Amorphous forms however perform best in both parameters as there is no lattice energy which needs to be overcome. Technical concepts for stabilisation of the amorphous state suffer from high energy inputs (e.g. melt extrusion with polymers), which may cause side reactions and degradation of the poorly water soluble compound. In other cases the mechanical properties of the product are poor as they are waxy solids or viscous liquids. Porous particles are also used to interrupt crystallisation due to interactions with the surface of the particle. Obviously this method leads to small mounts of loading, which is also a disadvantage for further applications. The choice of appropriate carrier, matrix materials is therefore crucial for practical considerations. Takeuchi et al. (Int. J. Pharm. 293 (2005) 155-164) describe the use of porous silica for the formation of solid dispersion particles with Indomethacin (IMC).
WO 2005/051358 A1 describes pharmaceutical compositions comprising porous particle carriers and a water-soluble polymer for increasing the bioavailability of water-insoluble drugs. EP 0 454 044 B1 describes pharmacological compositions comprising polyelectrolyte complexes in microparticulate form.

### Summary of the invention

The present invention relates to the stabilization of amorphous states of drugs using sponge-like carrier matrices. Amorphous states of drugs herein relate to states of drugs that lack crystalline structure or that have a very small portion of residual crystalline structure, preferably below about 15 %, more preferably below about 10 %, even more preferably below about 1 % and most preferably no residual crystalline structure. The degree of amorphous states and crystalline structure in a sample of a drug or active ingredient can be determined using X-ray powder diffraction (XRPD). Pharmaceutical compositions and methods for their production are provided by the present invention. The sponge-like carrier matrices of the pharmaceutical compositions of the invention can be used as scaffold for the deposition of poorly soluble drugs in the amorphous state.

These compositions can be used for the stabilisation of drugs in the amorphous state, for rheology improvement and for solubilisation enhancement or combinations thereof The sponge-like carrier matrix mentioned above may comprise polyelectrolyte complexes (PEC) formed by the electrostatic interaction between a polyanion and a polycation. The assembly mentioned above may also comprise inorganic carriers.
These carriers can be loaded with an active compound, combinations of excipient and active ingredients or combinations of polyelectrolytes and active ingredients provided that excipient or polyelectrolyte and active ingredient are soluble or molecular dispersed in the same solvent or solvent mixture. The scaffold (i.e. the carrier) itself may also be formed during the loading process with the active ingredient.

The present invention relates to a pharmaceutical composition comprising
a) an active ingredient,
b) a sponge-like carrier matrix, and
c) optionally at least one excipient.

In a first particular aspect the present invention relates to a pharmaceutical composition comprising
a) an active ingredient,
b) at least one excipient, and
c) a polyelectrolyte complex as a carrier.

The polyelectrolyte complex preferably comprises
i) a polyanion or an ampholyte with overall negative charge at a pH above the isoelectric point, and
ii) a polycation or an ampholyte with overall positive charge at a pH below the isoelectric point.

In a second particular aspect, the present invention relates to a pharmaceutical composition comprising
a) an active ingredient,
b) a porous particles as a carrier, and
c) optionally at least one excipient.

This second aspect provides a carrier, particularly porous particles, with a high inner surface.

In one particularly preferred embodiment of the second aspect of the present invention, the pharmaceutical composition comprises one or more low molecular weight excipients.

In another particularly preferred embodiment of the second aspect of the present invention, the pharmaceutical composition comprises no further low or high molecular weight excipients.

The present invention also relates to a method for producing a pharmaceutical composition according to the first aspect of the pharmaceutical composition, comprising the steps of
a.) dissolving an active ingredient and optionally a further excipient in an organic solvent,
b.) adding said organic solvent comprising the active ingredient to a dry polyelectrolyte complex.
c.) optionally removing the organic solvent.

The polyelectrolyte complex can be prepared by a method comprising the steps of
a.) mixing a polyanionic compound and a polycationic compound, wherein the polyanionic compound and/or the polycationic compound are dissolved in an aqueous solution or in powder form,
b.) wetting the mixture with water if the polycationic compound and the polyanionic compound are in powder form to form a slurry, and
c.) drying the mixture by freeze-drying, by spray-drying, by evaporation in a rotary evaporator, by heating, or by applying a vacuum or combinations thereof

The present invention relates to a method for producing a pharmaceutical composition according to the second aspect of the pharmaceutical composition, comprising the steps of
a.) dissolving an active ingredient and optionally a further excipient in an organic solvent,
b.) adding said organic solvent comprising the active ingredient to porous particles comprising a carrier material selected from the group comprising magnesium aluminometasilicate (MAS), anhydrous dibasic calcium phosphate, microcrystalline cellulose, cross-linked sodium carboxymethyl cellulose, soy bean hull fiber, and agglomerated silicon dioxide.
c.) optionally removing the organic solvent.

The present invention also relates to a pharmaceutical composition obtainable by any of the methods of the present invention.

### Detailed description of the invention

The present invention relates to a pharmaceutical composition comprising
a) an active ingredient,
b) a sponge-like carrier matrix , and
c) optionally at least one excipient.

The sponge-like carrier matrix herein alone or together with the excipient stabilises the amorphous active ingredient (i.e. the drug, particularly drugs with a low solubility in water), thereby facilitating the delivery of the active ingredient to its target and enhancing the bioavailability as compared to the unformulated drug substance or compared to existing carrier systems. The present invention relates in a first aspect to a pharmaceutical composition as defined above, wherein the sponge-like carrier matrix comprises polyelectrolyte complexes. In a second aspect the present invention relates to a pharmaceutical composition as defined above, wherein the sponge-like carrier matrix comprises porous particles. Porous particles are preferably microparticles with pores and/or holes. The have preferably dimensions in the µm range, preferably in the range of from about 1 to 700 µm, more preferably 1 to 250 µm, even more preferably 1 to 250 µm and most preferably 75 to 250 µm. The sponge-like, cavernous carrier matrices are preferably in form of supramolecular structures, particles, flakes and/or bricks (e.g. amorphous matrices of brittle nature). The sponge-like carrier matrices described herein preferably improve the rheology of the drug formulation.

The optional excipient may be a low molecular weight excipient or a high molecular weight excipient. A low molecular weight excipient herein refers to an excipient with a molecular mass of below about 1500 Da, preferably below 1400 Da, more preferably below 1200 Da, more preferably below 1000 Da, more preferably below 800 Da, even more preferably below 750 Da and most preferably below 500 Da. A high molecular weight excipient herein refers to an excipient with a molecular mass of about 1500 Da or above 1500 Da. A polymeric excipient comprises repetitive covalent bounds between one or more monomers. Typically each monomer is present multiple times in the polymer in a statistical or block wise manner. Therefore polymers typically belong to the class of high molecular weight excipients as defined above. Synthetic polymers are synthesized using polymerisation reactions, in which the product has no uniform molecular weight, but a molecular weight distribution. In contrast, natural polymers such as proteins and nucleic acids usually have a defined structure and molecular weight. In the context of the second aspect of the invention (i.e. the pharmaceutical composition comprising porous particles), the excipient is preferably a low molecular weight excipient.

Low molecular weight excipient are for example selected from the group comprising sorbitan monopalmitates (SPAN), polysorbates (TWEEN^{®}), surfactants in general, polyalcohols as e.g. glycerole, manniole, monosaccharides (e.g. glucose, lactose), amino acid and peptide. Preferred polysorbates are selected from the group comprising TWEEN^{®} 80, TWEEN^{®} 65, TWEEN^{®} 60, TWEEN^{®} 40 and TWEEN^{®} 20. The low molecular weight excipient may be a permeation enhancer or a solubiliser.

High molecular weight excipient are for example selected from the group comprising poly(vinylpyrrolidone) (PVP), poly(ethyleneglycole) (PEG), poly(propylenglycole) (PPG), polyvinyl alcohol, Eudragit E, and Eudragit S, polysaccharide such as dextran, starch, cellulose derivatives or chitosan, polypeptide such as poly-lysine or protein such as albumin, e.g. human serum albumin (HSA).

The excipient may in one embodiment be a monomer or in another embodiment be a polymer comprising repetitive structural units.

In some particular embodiments the excipient is insoluble in water. In further embodiments the excipient is insoluble in water but soluble in diluted acids (preferably with a pH below about 4.5) or diluted bases (preferably with a pH above about 8).

It is preferred that the ratio between the low molecular weight excipient or the high molecular weight excipient with respect to the amount of drug in the pharmaceutical composition is in the range of from about 0 to 50 % (w/w), preferably below 50% (w/w), more preferably below 20% (w/w) and most preferably below 5% (w/w).

The active ingredient in the context of the present invention is preferably a low solubility drug according to groups II or IV of the *Biopharmaceutics Classification System* (BCS) (FDA). Low solubility drugs are all drugs that are not high solubility drugs according to the following definition of the FDA. A drug substance is considered highly soluble when the highest dose strength is soluble in ≤ 250 ml water over a pH range of 1 to 7.5.

The active ingredient may for example be selected from the group comprising
- Atorvastatin, Amiodarone, Candesartan-Cilexetil, Carvedilol, Clopidogrel bisulfate, Dipyridamole, Eprosartan mesylate, Epierenone, Ezetimibe, Felodipine, Furosemide, Isradipine, Lovastatin, Metolazone, Nicardipine, Nisoldipine Olmesartan medoxomil, Propafenone HCl, Qinapril, Ramipril, Simvastatin, Telmisartan, Trandolapril, Valsartan and other cardio-vascular active drugs;
- Acyclovir, Adefovir, Dipivoxil, Amphotericin, Amprenavir, Cefixime, Ceftazidime, Clarithromycin, Clotrimazole, Efavirenz, Ganciclovir, Itraconazole, Norfloxacin, Nystatin Ritonavir, Saquinavir and other anti-infective drugs including anti-bacterial, anti fungal and anti-parasitic drugs;
- Cisplatin, Docetaxel, Etoposide, Exemestane, Idarubicin, Irinotecan, Melphalan, Mercaptopurine, Mitotane, Paclitaxel, Valrubicin and other drugs used in oncology;
- Azathioprine, Tacrolimus, Cyclosporine, Pimecrolimus, Sirolimus and other immonosupressive drugs;
- Clozapine, Entacapone, Fluphenazine, Imipramine, Nefazodone, Olanzapine, Paroxetine, Pimozide, Sertraline, Triazolam, Zaleplon, Ziprasidoneand and other drugs for CNS indications;
- Danazol, Dutasteride, Medroxyprogesterone, Raloxifene, Sildenafil, Tadalafil, Testosterone, Vardenafil and other drugs used for reproductive health;
- Celecoxib, Dihydroergotamine Mesylate, Eletriptan, Ergoloidmesylates, Ergotamine-tartrate, Nabumetone and other drugs against arthritis and pain;
- Bosentan, Budesonide, Desloratadine, Fexofenadin, Fluticasone, Loratadine, Mometasone, Salmeterol Xinafoate, Triamcinolon Acetonide, Zafirlukast and other drugs for respiratory indications; and
- Dronabinol, Famotidine, Glyburide, Hyoscyamine, Isotretinoin, Megestrol, Mesalamine, Modafinil, Mosapride, Nimodipine, Perphenazine, Propofol, Sucralfate, Thalidomide, Trizanidine hydrochloride and other drugs for various indications including in particular gastro-intestinal disorders, diabetes and dermatology indications.

Preferred active ingredients of the above list that do not form salts at any pH.

In preferred embodiments of the invention, the concentration of the active ingredient in the pharmaceutical composition is in the range of from 0 to 90% (w/w), preferably 0 to 50 %, more preferably between about 5 and 25% (w/w), even more preferably in the range of from about 10 to 20% (w/w) and most preferably between about 8 and 20 % (w/w). All concentration percentages herein refer to weight-per-weight (w/w) unless otherwise stated. (w/v) relates to weight-per volume.

Preferably, the active ingredient is located on the inner surfaces of the porous particles of the pharmaceutical composition and not on the outer surface.

Preferably, residual organic solvents, particularly class 3 solvents (solvent with low toxic potential) as defined in the The European Pharmacopoeia (Ph. Eur.) 5 (EDQM 2007), paragraph 5.4 solvents, are present at a concentration of less than 5 %, less than 4 %, less than 3 %, less than 2 % or less than 1 % (w/w).

The pharmaceutical composition of the present invention is not limited to a particular way of administration. The administration may for example be an intramuscular, subcutaneous, parenteral, ophthalmic or oral administration.

As mentioned above, in the first aspect the present invention relates to a pharmaceutical composition comprising
a) an active ingredient,
b) at least one excipient, and
c) a polyelectrolyte complex as a carrier.

The polyelectrolyte complex preferably comprises
i) a polyanion or an ampholyte with overall negative charge at a pH above the isoelectric point, and
ii) a polycation or an ampholyte with overall positive charge at a pH below the isoelectric point.

Polyelectrolytes are polymers whose repeating units, or at least one of the repeating units, bear an electrolyte group. An electrolyte is any substance dissociating in an appropriate medium (e.g. water) into ions of opposite charge. A polymer is a macromolecule composed of repeating structural units connected by covalent chemical bonds. There are heteropolymers (comprising two or more kinds of structural units) and homopolymers (comprising only one kind of structural units). A polyelectrolyte may contain negatively and positively charge groups. These polyelectrolytes are called ampholytes. A skilled person knows that the net charge of an ampholytic compound in aqueous solutions depends on its isoelectrical point and on the pH of the solution. A polycation has an overall positive charge and comprises structural units with a net positive charge, whereas polyanions have an overall negative charge and comprises structural units with a net negative charge. A polyelectrolyte may also be considered as comprising a polyacid and a polybase.

The polyanion is preferably selected from the group comprising xylan polysulfate, dextran sulfate, poly(amino acids) such as polyaspartic acid or polyglutamic acid, polysaccharide polysulfate such as sulfate of starch hydrolysate, inulin, hydroxyethylstarch, polysaccharide polysulfonate, polysaccharide polyphosphate, carboxymethylcellulose, gelatin B, collagen, HSA (Human Serum Albumin) or other albumins, Eudragit S and polyphosphates.

The polycation is preferably selected from the group comprising poly-L-lysine, poly-α, β-(2-dimethylaminoethyl)-D,L-aspartamide, chitosan, lysine octadecyl ester, aminated dextran, laminated cyclodextrin, aminated cellulose ether, protamine (sulfate), gelatin A, Eudragit E, HSA (Human Serum Albumin) or other albumins, casein, nucleic acid (e.g. DNA, RNA, LNA or PNA) and aminated pectin.

Preferred polyelectrolyte combinations in the context of the first aspect of the present invention are:
a.) protalmine/carboxylnetliyl cellulose: particularly as carrier for intramuscular, subcutaneous, parenteral ophthalmic and oral administration
b.) protamine/gelatine B: particularly as carrier for intramuscular, subcutaneous, parenteral, ophthalmic and oral administration
c.) HSA/gelatine: as universal carrier
d.) Eudragit E/Eudragit S: particularly as carrier for oral administration
e.) casein/polyanion: particularly as carrier for oral administration

The polyelectrolyte complex may additionally comprise inorganic or organic ions and/or salts, e.g. sodium chloride and antifoam agents, such as polypropylene glycol, polyethylene glycol, polyvinyl alcohol and non-complexed polymers.

In the second aspect, the present invention relates to a pharmaceutical composition comprising
a) an active ingredient,
b) a porous particles as a carrier, and
c) optionally at least one excipient.

In one particularly preferred embodiment of the second aspect of the present invention, the pharmaceutical composition comprises one or more low molecular weight excipients.

In another particularly preferred embodiment of the second aspect of the present invention, the pharmaceutical composition comprises no further low or high molecular weight excipients.

The porous particles preferably comprise a carrier material selected from the group comprising magnesium aluminometasilicate (MAS), anhydrous dibasic calcium phosphate, microcrystalline cellulose, cross-linked sodium carboxymethyl cellulose, soy bean hull fiber, and agglomerated silicon dioxide.

Magnesium aluminometasilicate is available from Fuji Chemicals Co. udder the name Neusilin®. Different forms of Neusilin® are available. However, the preferred magnesium aluminometasilicate preparation in the context of the present invention is Neusilin® US2. Unless otherwise stated, "Neusilin®" and "Neusilin® US" herein refers to Neusilin® US2, particularly in the examples.

Agglomerated silicon dioxide is available from Evonik under the trade name Aerosil®.

It has surprisingly been found in the present invention that the porous particles stabilize the amorphous drugs even without the addition of further high molecular weight excipients or without the addition of excipients at all. Further it has surprisingly been found that the polyelectrolyte complex carriers even stabilize active ingredients that do not form salts at any pH and/or that are low solubility drugs according to groups II or TV of the *Biopharmaceutics Classification System* (BCS) (FDA). The use of polyelectrolyte complexes enables new routes of administrations for such classes of active ingredients. The choice of the polyelectrolyte allows for a targeting of the active ingredient and for the use of a wide variety of active ingredients. Furthermore, the pharmaceutical compositions of the present invention enhance the bioavailability of the active ingredient.

The present invention also relates to methods for the production of the pharmaceutical compositions of the present invention. All definitions and embodiments, particularly those for the active ingredient, the excipient and the sponge-like matrix, also apply to the methods for the production of the pharmaceutical compositions.

The present invention relates to a method for producing a pharmaceutical composition according to the first aspect of the pharmaceutical composition, comprising the steps of
a.) dissolving an active ingredient and optionally a further excipient in an organic solvent,
b.) adding said organic solvent comprising the active ingredient to a dry polyelectrolyte complex,
c.) optionally removing the organic solvent.

Polyelectrolyte complexes build amorphous matrices, which depending on the production method and composition may vary in the rheological behaviour, particle size, degree of porosity, pore size and in their mechanical properties in general. These polyelectrolyte matrices, as well as other inorganic carriers, are optimal scaffolds for the precipitation of a drug, previously dissolved in an organic solvent, into the amorphous state. These compositions besides from stabilizing the drug in the amorphous state also have the additional role of being appropriate filler excipients for solid dosage form development.

The polyelectrolyte complex can be prepared by a method comprising the steps of
a.) mixing a polyanionic compound and a polycationic compound, wherein the polyanionic compound and/or the polycationic compound are dissolved in an aqueous solution or in powder form,
b.) wetting the mixture with water if the polycationic compound and the polyanionic compound are in powder form to form a slurry, and
c.) drying the mixture by freeze-drying, by spray-drying, by evaporation in a rotary evaporator, by heating, or by applying a vacuum or combinations thereof

Particular methods for the production of polyelectrolyte complexes are illustrated in examples 1 to 8. The solutions comprising the polyanionic or polycationic compounds may additionally comprise salts, e.g. sodium chloride, and preferably have ion strengths of in the range of from about 0 to 1 M, preferably in the range of from about 0.01 M to about 0.125 M. The polyelectrolyte may preferably have concentrations in the aqueous solution in a range in which the viscosity is not limiting the processability, preferably from about 1 to 2% (w/v).

The present invention relates to a method for producing a pharmaceutical composition according to the second aspect of the pharmaceutical composition, comprising the steps of
a.) dissolving an active ingredient and optionally a further excipient in an organic solvent,
b.) adding said organic solvent comprising the active ingredient to porous particles comprising a carrier material selected from the group comprising magnesium aluminometasilicate (MAS), anhydrous dibasic calcium phosphate, microcrystalline cellulose, cross-linked sodium carboxymethyl cellulose, soy bean hull fiber, and agglomerated silicon dioxide.
c.) optionally removing the organic solvent.

Mixing may for example be performed by stirring, vortexing, wet-milling or using a homogenize.

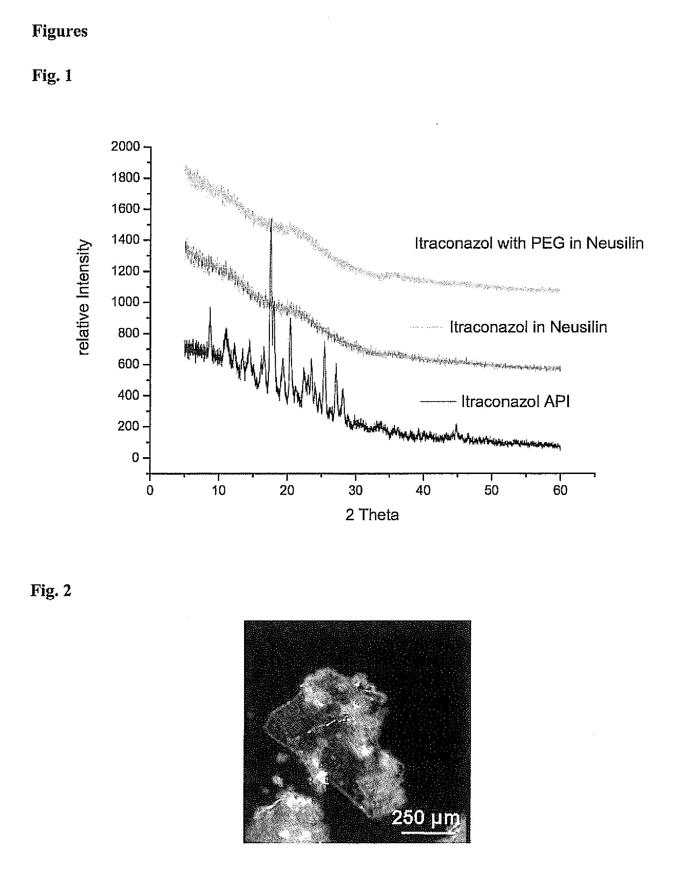

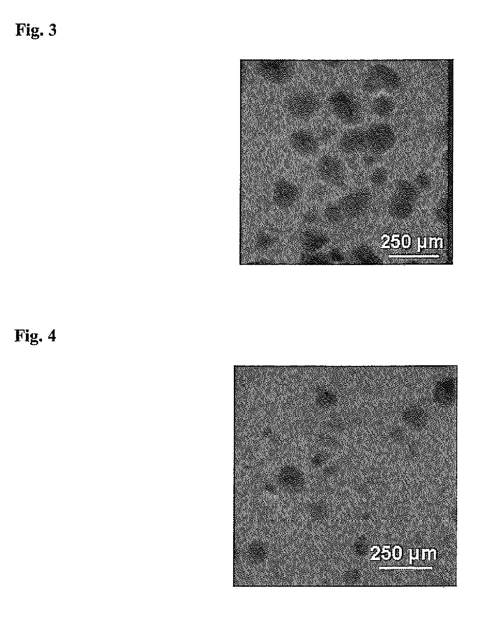

Particular methods for the production of a pharmaceutical composition according to the second aspect are illustrated in examples 9 and 10 (Itraconanzole with Neusilin®) and examples 12 to 15 (Candesartan with Neusilin®) (also shown in polarisation microscopy images in Figs. 3, 4, and 5). Example 11 shows in comparison a pharmaceutical composition without Neusilin® (also shown in a polarisation microscopy image in Fig. 2).

The resulting pharmaceutical composition preferably comprises porous microparticles that do not form superparticular structures or complexes. Further the method preferably results in porous particles in which the active ingredient (and optionally the excipient) is located on the inner surface of the particles and not on the outer surface.

During the method, the organic solvent comprising the active ingredient (and optionally the excipient) is preferably completely soaked into the porous particles. Furthermore, the method preferably results in amorphous particles with little to no residual crystal-like structure as judged from X-ray powder diffraction spectra.

The organic solvent in the context of the methods of this invention is preferably a class 3 solvent (solvent with low toxic potential) as defined in the The European Pharmacopoeia (Ph. Eur.) 5 (EDQM 2007), paragraph 5.4, or is selected from the group comprising acetic acid, heptane, acetone, isobutyl acetate, anisole, isopropyl acetate, 1-butanol, methyl acetate, 2-butanol, 3-methyl-l-butanol, butyl acetate, methylethyl ketone, tert-butylznethyl ether, methylisobutyl ketone, cumene, 2-metliyl-1-propanol, dimethyl sulfoxide, pentanel, methanol, 1-pentanol, ethyl acetate, 1-propanol, ethyl ether, 2-propanol, ethyl formate, propyl acetate and formic acid or is a mixture of two or more organic solvents selected from said group. Strong limitations apply to the presence of residual non-class 3 solvents in the final pharmaceutical composition. Therefore such solvents are less preferred. After removal of the organic solvent, the amount residual solvent needs to be in accordance with regulatory requirements. However, in some embodiments, the solvent may be identical to the low molecular weight excipient. In this particular case the organic solvent must not be removed. In all other cases the solvent is preferably removed. A skilled person is aware of the melting temperatures of the low molecular weight excipient above which it can act as a solvent for the active ingredient.

The active ingredients may preferably have concentrations in the organic solvent of more than about 20mg/ml, more preferably more than about 50 mg/ml, most preferably more than about 100mg/ml.

The low or high molecular weight excipient may preferably have concentrations in the organic solvent of more than about 2 mg/ml, more preferably more than about 15 mg/ml, most preferably more than about 25 mg/ml.

The organic solvent may for example be removed by freeze-drying, by spray-drying, by evaporation in a rotary evaporator, by heating, or by applying a vacuum or combinations thereon

In particular embodiments, the organic solvent comprising the active ingredient is added dropwise under permanent mixing to the polyelectrolyte complex or to the porous particles comprising the carrier material.

The present invention also relates to a pharmaceutical composition obtainable by any of the methods of the present invention. All definitions and embodiments, particularly those for the active ingredient, the excipient and the sponge-like matrix, as outlined above also apply to the pharmaceutical compositions obtainable by the methods for the production of the pharmaceutical compositions.

In particular, the present invention relates - according to the first aspect of the present invention - to a pharmaceutical composition obtainable by a method comprising the steps of
a.) dissolving an active ingredient and optionally a further excipient in an organic solvent,
b.) adding said organic solvent comprising the active ingredient to a dry polyelectrolyte complex.
c.) optionally removing the organic solvent.

The polyelectrolyte complex can be prepared by a method comprising the steps of
a.) mixing a polyanionic compound and a polycationic compound, wherein the polyanionic compound and/or the polycationic compound are dissolved in an aqueous solution or in powder form,
b.) wetting the mixture with water if the polycationic compound ailed the polyanionic compound are in powder form to form a slurry, and
c.) drying the mixture by freeze-drying, by spray-drying, by evaporation in a rotary evaporator, by heating, or by applying a vacuum or combinations thereof

The solutions comprising the polyanionic or polycationic compounds may additionally comprise salts, e.g. sodium chloride, and preferably have ion strengths of in the range of from about 0 to 1 M, preferably in the range of from about 0.01 M to about 0.125 M. The polyelectrolyte may preferably have concentrations in the aqueous solution in a range in which the viscosity is not limiting the processability.

According to the second aspect of the present invention, the invention relates to a pharmaceutical composition obtainable by a method comprising the steps of
a.) dissolving an active ingredient and optionally a further excipient in an organic solvent,
b.) adding said organic solvent comprising the active ingredient to porous particles comprising a carrier material selected from the group comprising magnesium aluminometasilicate (MAS), anhydrous dibasic calcium phosphate, microcrystalline cellulose, cross-linked sodium carboxymethyl cellulose, soy bean hull fiber, and agglomerated silicon dioxide.
c.) optionally removing the organic solvent.

The organic solvent in the context of the pharmaceutical compositions obtainable by the methods of this invention is preferably a class 3 solvent (solvent with low toxic potential) as defined in the The European Pharmacopoeia (Ph. Eur.) 5 (EDQM 2007), paragraph 5.4, or is selected from the group comprising acetic acid, heptane, acetone, isobutyl acetate, anisole, isopropyl acetate, 1-butanol, methyl acetate, 2-butanol, 3-methyl-1-butanol, butyl acetate, methylethyl ketone, tert-butylmethyl ether, methylisobutyl ketone, cumene, 2-methyl-1-propanol, dimethyl sulfoxide, pentanel, ethanol, 1-pentanol, ethyl acetate, 1-propanol, ethyl ether, 2,-propanol, ethyl formate, propyl acetate and formic acid or is a mixture of two or more organic solvents selected from said group. Strong limitations apply to the presence of residual non-class 3 solvents in the final Pharmaceutical composition. Therefore such solvents are less preferred. After removal of the organic solvent, the amount residual solvent needs to be in accordance with regulatory requirements. However, in some embodiments, the solvent may be identical to the low molecular weight excipient. In this particular case the organic solvent must not be removed. In all other cases the solvent is preferably removed. A skilled person is aware of the melting temperatures of the low molecular weight excipient above which it can act as a solvent for the active ingredient.

The active ingredients may preferably have concentrations in the organic solvent of more than about 20mg/ml, more preferably more than about 50 mg/ml, most preferably more than about 100mg/ml.

The low or high molecular weight excipient may preferably have concentrations in the organic solvent of more than about 2 mg/ml, more preferably more than about 15 mg/ml, most preferably more than about 25 mg/ml.

The organic solvent may for example be removed by freeze-drying, by spray-drying, by evaporation in a rotary evaporator, by heating, or by applying a vacuum or combinations thereof

In particular embodiments, the organic solvent comprising the active ingredient is added dropwise under permanent mixing to the polyelectrolyte complex or to the porous particles comprising the carrier material.

The following examples illustrate particular embodiments and aspects of the present invention. However, they are not limiting the scope of the invention.

Examples 1 to 8 illustrate methods for the production of polyelectrolyte complexes.

Examples 9 and 10 illustrate amorphous formulations of Itraconazole.

Examples 11 to 18 illustrate amorphous formulations of Candesartan cilexetil.

### Examples

### Example 1

### Production of polyelectrolyte complexes

A solution containing 2% (w/v) protamine sulfate with an ionic strength of 0.01 M was added to a solution containing 2% (w/v) carboxymethylcellulose with an ionic strength of 0.01 M under mixing with an ultra-turrax®. The resulting suspension was lyophilised and stored at RT until further use.

### Example 2

### Production of polyelectrolyte complexes

A solution containing 2% (w/v) protamine sulfate with an ionic strength of 0.125 M was added to a solution containing 2% (w/v) carboxymethylcellulose with an ionic strength of 0.125 M under mixing with an ultra-turrax®. The resulting suspension was lyophilised and stored at RT until further use.

### Example 3

### Production of polyelectrolyte complexes

A solution containing 2% (w/v) protamine sulfate with an ionic strength of 0.125 M was added to a solution containing 1% (w/v) carboxymethylcellulose with an ionic strength of 0.125 M under mixing with an ultra-turrax. The resulting suspension was lyophilised and stored at RT until further use.

### Example 4

### Production of polyelectrolyte complexes

A solution containing 2% (w/v) protamine sulfate with an ionic strength of 0.125 M and a concentration of 1% (w/v) of PPG was added to a solution containing 2% (w/v) carboxymethylcellulose with an ionic strength of 0.125 M and 1% (w/v) PPG under mixing with an ultra-turrax®. The resulting suspension was lyophilised and stored at RT until further use.

### Example 5

### Production of polyelectrolyte complexes

A solution containing 2% (w/v) protamine sulfate with an ionic strength of 0.01 M and a concentration of 1% (w/v) of PPG was added to a solution containing 2% (w/v) carboxymethylcellulose with an ionic strength of 0.01 M and 1% (w/v) PPG under mixing with an ultra-turrax®. The resulting suspension was lyophilised and stored at RT until further use.

### Example 6

### Production of polyelectrolyte complexes

A solution containing 2 % (w/v) protamine sulfate with an ionic strength of 0.01 M and a concentration of 1% (w/v) of PPG was added to a solution containing 1% (w/v) carboxymethylcellulose with an ionic strength of 0.01 M and 1% (w/v) PPG under mixing with an ultra-turrax®. The resulting suspension was lyophilised and stored at RT until further use.

### Example 7

### Production of polyelectrolyte complexes

A solution containing 2% (w/v) protamine sulfate with an ionic strength of 0.125 M and a concentration of 1% (w/v) of PPG was added to a solution containing 1% (w/v) carboxymethylcellulose with an ionic strength of 0.125 M and 1% (w/v) PPG under mixing with an ultra-turrax. The resulting suspension was lyophilised and stored at RT until further use.

### Example 8

### Production of polyelectrolyte complexes

A solution containing 2% (w/v) protamine sulfate with an ionic strength of 0,125 M and a concentration of 1% (w/v) of PPG was added to a solution containing 2% (w/v) carboxymethylcellulose with an ionic strength of 0.125 M and 1% (w/v) PPG under mixing with an ultra-turrax®. The resulting suspension was lyophilised and stored at RT until further use.

### Example 9

### Loading of Neusilin® with Itraconazole

A Solution of 500 mg Itraconazole in 2.5 ml of dichloromethane was prepared. This clear solution was added dropwise to 2.5g Neusilin® US under permanent mixing. If agglutination was observed mixing was continued until a fine powder was achieved, only than the next drop of solution was added. The solvent was removed using a rotary evaporator. The amount of total Itraconazole was analysed using HPLC and found to be 17% (w/w). The formulation was analysed using x-ray powder diffraction (XRPD) measurements. No crystalline material was found, even about 1 year after preparation. See appended Fig. 1.

### Example 10

### Loading of Neusilin® with Itraconazole and PEG

A Solution of 500 mg Itraconazole and 200 mg PEG in 2.5 ml of dichloromethane was prepared. This clear solution was added dropwise to 2.5g Neusilin® US under permanent mixing. If agglutination was observed mixing was continued until a fine powder was achieved, only than the next drop of solution was added. The solvent was removed using a rotary evaporator. The amount of total Itraconazole was analysed using HPLC and found to be 16% (w/w). The formulation was analysed using XRPD measurements. No crystalline material was found even about 1 year after preparation. See appended Fig. 1.

### Example 11

A Solution of 20mg Eudragid E (EuE) and 200mg candesartan-cilextil in 4ml acetone was prepared. This clear solution was transferred to a rotary evaporator and the solvent was removed. The respective residue was examined with polarisation microscopy (see appended Fig. 2). Here high intensity spots are related to crystalline material.

### Example 12

A Solution of 50mg Eudragid E and 100mg candesaitan-cilextil in 1ml acetone was prepared. This clear solution was added dropwise to 0.5g Neusilin® US under permanent mixing. If agglutination was observed mixing was continued until a fine powder was achieved, only than the next drop of solution was added. A polarisation microscopy image showed no evidence for crystalline materials (see appended Fig. 3).

### Example 13

A Solution of 50mg Eudragid S (EuS) and 100mg candesartan-cilextil in 1ml acetone and 0.5 ml 2-propanol was prepared. This clear solution was added dropwise to 0.5g Neusilin® US under permanent mixing. If agglutination was observed mixing was continued until a fine powder was achieved, only than the next drop of solution was added. A polarisation microscopy image showed no evidence for crystalline materials (see appended Fig. 4).

### Example 14

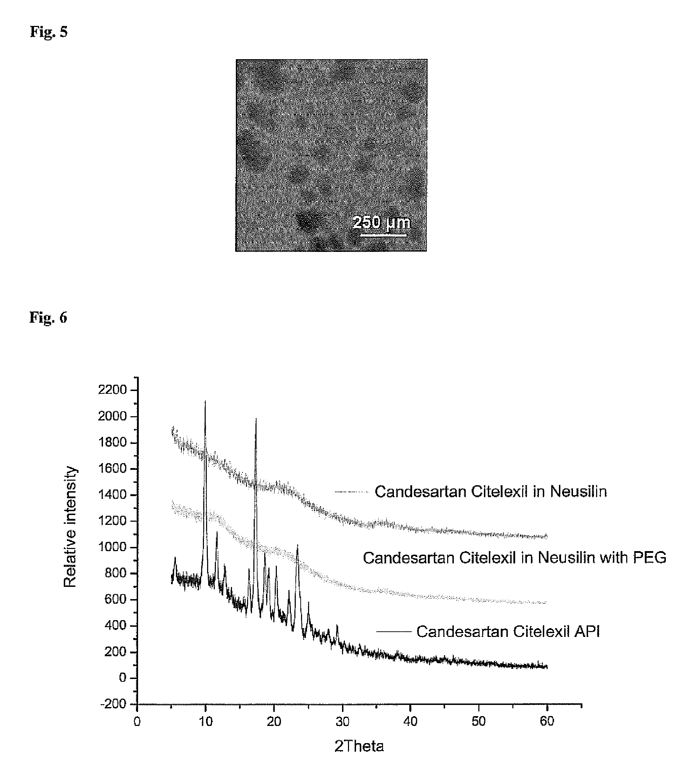

A Solution of 50mg poly(ethylene glycol) (PEG) and 100mg candesartan-cilextil in 1m! dichloromethane was prepared. This clear solution was added dropwise to 0.5g Neusilin® US under permanent mixing. If agglitunation was observed mixing was continued until a fine powder was achieved, only than the next drop of solution was added. A polarisation microscopy image showed no evidence for crystalline materials (see appended Fig. 5). As well an XRPD study was performed, proving no crystalline materials could be found in the formulation (see appended Fig. 6).

### Example 15

A Solution of 30mg candesartan-cilextil in 300ml dichloromethane was prepared. This clear solution was added dropwise to 0.15g Neusilin® US under permanent mixing. If agglutination was observed mixing was continued until a fine powder was achieved, only than the next drop of solution was added. In a XRPD study no crystalline materials could be found in the formulation (see appended Fig. 6),

### Example 16

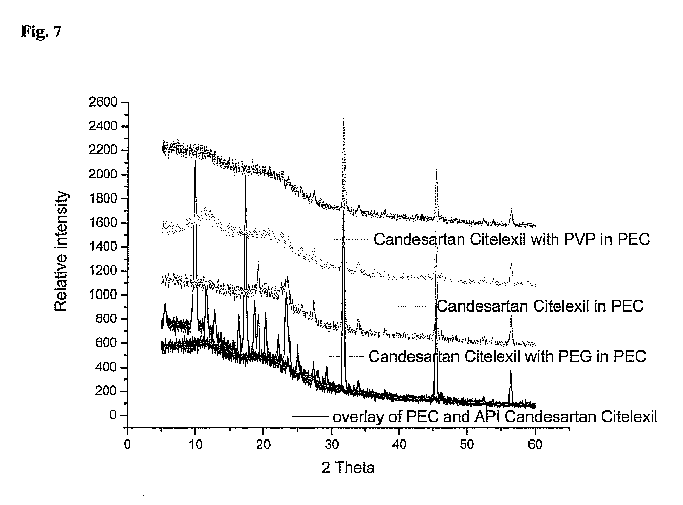

A Solution of 20mg candesartan-cilextil in 200ml dichloromethane was prepared. This clear solution was added dropwise to 0.1g polyelectrolyte complex (PEC) under permanent mixing. If agglutination was observed mixing was continued until a fine powder was achieved, only than the next drop of solution was added. In a XRPD study only weak reflexes were found (see appended Fig. 7).

### Example 17

A Solution of 20mg candesartan-cilextil and 10 mg PEG in 200ml dichloromethane was prepared. This clear solution was added dropwise to 0.1g PEC under permanent mixing. If agglutination was observed mixing was continued until a fine powder was achieved, only than the next drop of solution was addled, In a XRPD study only weak reflexes were found (see appended Fig. 7).

### Example 18

A Solution of 20mg candesartan-cilextil and 10 PVP in 200 ml dichloromethane was prepared. This clear solution was added dropwise to 0.1g PEC under permanent mixing. If agglutination was observed mixing was continued until a fine powder was achieved, only than the next drop of solution was added. In a XRPD study only very weak reflexes were found (see appended Fig. 7).

### Description of drawings

Fig. 1 shows the results of an x-ray powder diffraction analysis (XRPD) of Neusilin/Itraconazole formulations with (example 10) and without PEG (example 9) in comparison to the powder spectrum of Itraconazole. None of the characteristic peaks of the plain substance can be seen in the spectrum of the loaded matrix. This shows that the material is truly amorphous. An arbitary constant value was added to the latter spectra to enhance visibility.
Fig. 2 shows a polarisation microscopy image of the composition of example 11; high intensity reflexes due to crystalline material.
Fig. 3 shows a polarisation microscopy image of the composition of example 12; no evidence for crystalline material visible. Crystalline material would give rise to high intensity reflexes as seen in Fig. 2. The round particles are Neusilin® particles.
Fig. 4 shows a polarisation microscopy image of the composition of example 13; no evidence for crystalline material visible. Crystalline material would give rise to high intensity reflexes as seen in Fig. 2. The round particles are Neusilin® particles.
Fig. 5 shows a polarisation microscopy image of the composition of example 14; no evidence for crystalline material visible. Crystalline material would result in high intensity reflexes as seen in Fig. 2. The round particles are Neusilin® particles.
Fig. 6 shows the results of an x-ray powder diffraction analysis (XRPD) of Neusilin/candesartan-cilextil formulations with (example 15) and without PEG (example 14) in comparison to the powder spectrum of candesartan-cilextil. None of the characteristic peaks of the plain substance can be seen in the spectrum of the loaded matrix. This shows the material is truly amorphous. An arbitary constant value was added to the latter spectra to enhance visibility.
Fig. 7 shows the results of an x-ray powder diffraction analysis (XRPD) of polyelectrolyte complex/candesartan-cilextil formulations with PEG (example 17), with PVP (example 18) and without further excipient (example 16) in comparison to the powder spectrum of candesartan-cilextil. The characteristic peaks of the plain substance can be seen just slightly in the spectrum of the loaded matrix. The low peak intensity shows an high amount of amorphous active component in the matrix. An arbitary constant value was added to the latter spectra to enhance visibility.

## Claims

1. Pharmaceutical composition comprising
a) an active ingredient,
b) a sponge-like carrier matrix, and
c) optionally at least one excipient.

2. Pharmaceutical composition according to claim 1, wherein the excipient is a low molecular weight excipient with a molecular mass of below about 1500 Da or alternatively wherein the excipient is a high molecular weight excipient with a molecular mass of about 1500 Da or above 1500 Da.

3. Pharmaceutical composition according to claim 1 or 2, wherein the sponge-like carrier matrix comprises a polyelectrolyte complex or porous particles.

4. Pharmaceutical composition according to any one of the claims 1 to 3, wherein the low molecular weight excipient is selected from the group comprising sorbitan monopalmitates (SPAN), polysorbates (TWEEN), glycerole, polyalcohol, monosaccharides, amino acids and peptides or the high molecular weight excipient is selected from the group comprising poly(vinylpyrrolidone), poly(ethyleneglycole), poly(propylenglycole), polyvinyl alcohol, Eudragit E, Eudragit S, polypeptide, polysaccharide and protein.

5. Pharmaceutical composition according to any one of the claims 1 to 4, wherein the concentration of the low molecular weight excipient or the high molecular weight excipient in the pharmaceutical composition is in the range of from about 0 to 50 % (w/w).

6. Pharmaceutical composition according to any one of the claims 1 to 5, wherein the active ingredient is a low solubility drug according to groups II or IV of the Biopharmaceutics Classification System (BCS).

7. Pharmaceutical composition according to any one of the claims 1 to 6, wherein the active ingredient is selected from the group comprising Atorvastatin, Amiodarone, Candesartan-Cilexetil, Carvedilol, Clopidogrel bisulfate, Dipyridamole, Eprosartan mesylate, Epierenone, Ezetimibe, Felodipine, Furosemide, Isradipine, Lovastatin, Metolazone, Nicardipine, Nisoldipine Olmesartan medoxomil, Propafenone HCI, Qinapril, Ramipril, Simvastatin, Telmisartan, Trandolapril, Valsartan, Acyclovir, Adefovir, Dipivoxil, Amphotericin, Amprenavir, Cefixime, Ceftazidime, Clarithromycin, Clotrimazole, Efavirenz, Ganciclovir, Itraconazole, Norfloxacin, Nystatin Ritonavir, Saquinavir, Cisplatin, Docetaxel, Etoposide, Exemestane, Idarubicin, Irinotecan, Melphalan, Mercaptopurine, Mitotane, Paclitaxel, Valrubicin, Azathioprine, Tacrolimus, Cyclosporine, Pimecrolimus, Sirolimus, , Clozapine, Entacapone, Fluphenazine, Imipramine, Nefazodone, Olanzapine, Paroxetine, Pimozide, Sertraline, Triazolam, Zaleplon, Ziprasidoneand, Danazol, Dutasteride, Medroxyprogesterone, Raloxifene, Sildenafil, Tadalafil, Testosterone, Vardenafil and other drugs used for reproductive health, Celecoxib, Dihydroergotamine Mesylate, Eletriptan, Ergoloidmesylates, Ergotamine-tartrate, Nabumetone, Bosentan, Budesonide, Desloratadine, Fexofenadin, Fluticasone, Loratadine, Mometasone, Salmeterol Xinafoate, Triamcinolon Acetonide, Zafirlukast, Dronabinol, Famotidine, Glyburide, Hyoscyamine, Isotretinoin, Megestrol, Mesalamine, Modafinil, Mosapride, Nimodipine, Perphenazine, Propofol, Sucralfate, Thalidomide and Trizanidine hydrochloride.

8. Pharmaceutical composition according to any one of the claims 1 to 7, wherein the concentration of the active ingredient in the pharmaceutical composition is in the range of from 0 to 90% (w/w).

9. Pharmaceutical composition according to any one of the claims 1 to 8, wherein said sponge-like carrier matrix comprises a polyelectrolyte complex and the polyelectrolyte complex comprises
a) a polyanion or an ampholyte with overall negative charge at a pH above the isoelectric point, and
b) a polycation or an ampholyte with overall positive charge at a pH below the isoelectric point.

10. Pharmaceutical composition according to claim 9, wherein the polyanion is selected from the group comprising xylan polysulfate, dextran sulfate, poly(alnilio acids), polysaccharide polysulfate, inulin, hydroxyethylstarch, polysaccharide polysulfonate, polysaccharide polyphosphate, carboxymethylcellulose, gelatin B, Eudragit S, collagen, albumin and polyphosphate.

11. Pharmaceutical composition according to claim 9 or 10, wherein the polycation is selected from the group comprising poly-L-lysine, poly-α,β-(2-dimetliylaminoethyl)-D,L-aspartamide, chitosan, lysine octadecyl ester, aminated dextran, aminated cyclodextrin, aminated cellulose ether, protamine (sulfate), gelatine A, Eudragit E, albumin, casein, nucleic acid and aminated pectin.

12. Pharmaceutical composition according to any one of the claims 1 to 8, wherein the porous particles comprise a carrier material selected from the group comprising magnesium aluminometasilicate (MAS), anhydrous dibasic calcium phosphate, microcrystalline cellulose, cross-linked sodium carboxymethyl cellulose, soy bean hull fiber, and agglomerated silicon dioxide.

13. Method for producing a pharmaceutical composition according to claim 12, comprising the steps of
a) dissolving an active ingredient and optionally a further excipient in an organic solvent,
b) adding said organic solvent comprising the active ingredient to porous particles comprising a carrier material selected from the group comprising magnesium aluminometasilicate (MAS), anhydrous dibasic calcium phosphate, microcrystalline cellulose, cross-linked sodium carboxymethyl cellulose, soy bean hull fiber, and agglomerated silicon dioxide.
c) optionally removing the organic solvent.

14. Method for producing a pharmaceutical composition according to any one of the claims 9 to 11, comprising the steps of
a) dissolving an active ingredient and optionally a further excipient in an organic solvent,
b) adding said organic solvent comprising the active ingredient to a dry polyelectrolyte complex.
c) optionally removing the organic solvent.

15. Method according to claim 14, wherein the polyelectrolyte complex is prepared by a method comprising the steps of
a) mixing a polyanionic compound and a polycationic compound, wherein the polyanionic compound and/or the polycationic compound are dissolved in an aqueous solution or in powder form,
b) wetting the mixture with water if the polycationic compound and the polyanionic compound are in powder form to form a slurry, and
c) drying the mixture by freeze-drying, by spray-drying, by evaporation in a rotary evaporator, by heating, or by applying a vacuum or combinations thereof.

16. A Pharmaceutical composition obtainable by a method according to any one of the claims 13 to 15.
